# EUROPEAN PATENT SPECIFICATION

(11) **EP 3 472 134 B1**
(45) Date of publication and mention of the grant of the patent: **20.10.2021**
(21) Application number: 17733739.1
(22) Date of filing: 16.06.2017
(51) Int. Cl.: C07D 239/557, C07D 413/12

(54) **PROCESS FOR PREPARING COMPOUNDS USEFUL AS INTERMEDIATES FOR THE PREPARATION OF RALTEGRAVIR**
VERFAHREN ZUR HERSTELLUNG VON VERBINDUNGEN ALS ZWISCHENPRODUKTE ZUR HERSTELLUNG VON RALTEGRAVIR
PROCÉDÉ DE PRÉPARATION DE COMPOSÉS UTILISÉS COMME INTERMÉDIAIRES POUR LA PRÉPARATION DU RALTÉGRAVIR

(30) Priority: 21.06.2016 WO PCT/EP2016/001053; 02.02.2017 WO PCT/EP2017/025016; 06.02.2017 WO PCT/EP2017/025020
(43) Date of publication of application: 24.04.2019
(73) Proprietor: Pharmathen S.A., 15351 Pallini Attikis (GR)
(72) Inventor: KOFTIS, V., Theocharis, GR-57001 Thessaloniki (GR); NEOKOSMIDIS, Efstratios, GR-57001 Thessaloniki (GR); STATHAKIS, Christos, GR-57001 Thessaloniki (GR); GKIZIS, Petros, GR-57001 Thessaloniki (GR); ALEXANDRAKI, Elli, GR-57001 Thessaloniki (GR); TRAKOSSAS, Sakellarios, GR-57001 Thessaloniki (GR); TERZIDIS, Michael, GR-57001 Thessaloniki (GR); LITHADIOTI, Alexandra, GR-57001 Thessaloniki (GR); TSATSAS, Theodoros, GR-57001 Thessaloniki (GR); PANAGIOTIDIS, Theodoros, GR-57001 Thessaloniki (GR); VARVOGLI, Anastasia - Aikaterini, GR-57001 Thessaloniki (GR)
(86) International application number: PCT/EP2017/025167
(87) International publication number: WO 2017/220208

(56) References cited:
- US-A1- 2006 122 205
- BASSINDALE A R ET AL: "Chemoselective methylation of amides and heterocycles using chloromethyldimethylsilyl chloride", TETRAHEDRON LET, ELSEVIER, AMSTERDAM, NL, vol. 41, no. 25, 1 June 2000 (2000-06-01), pages 4933-4936, XP004205686, ISSN: 0040-4039, DOI: 10.1016/S0040-4039(00)00731-0

## Description

### TECHNICAL FIELD OF THE INVENTION

The present invention relates to a novel methylation process useful in the preparation of compounds which are intermediates in the synthesis of Raltegravir.

### BACKGROUND OF THE INVENTION

Raltegravir is an HIV-integrase inhibitor and finds its application as an antiretroviral drug, marketed in the form of its potassium salt under the brand name Isentress® by Merck & Co. (or Merck Sharp & Dome). The chemical name for Raltegravir potassium is 4-[N-(4-fluoro-benzyl)-carbamoyl]-1-methyl-2-{1-methyl-1-(5-methyl-1, 3, 4 oxadiazol-2-yl-carboxamido)ethyl}-6-oxo-1,6-dihydropyrimidin-5-olate potassium salt (Formula I). Raltegravir is disclosed in patent application WO2003035077.

WO2006060730 discloses a method for the preparation of Raltegravir (Scheme 1).

The methylation reaction onto a key intermediate is performed in the presence of magnesium methoxide, which allegedly favors the methylation on the ring nitrogen atom over the neighboring oxygen. The reaction however is not selective and treatment for the removal of the O-methylated isomer is required. According to the patent application, a mixed solvent system of methanol and t-butyl dimethyl ether is used for this purpose and still the product of the reaction contains "less than 0.5%" of the O-methylated product.

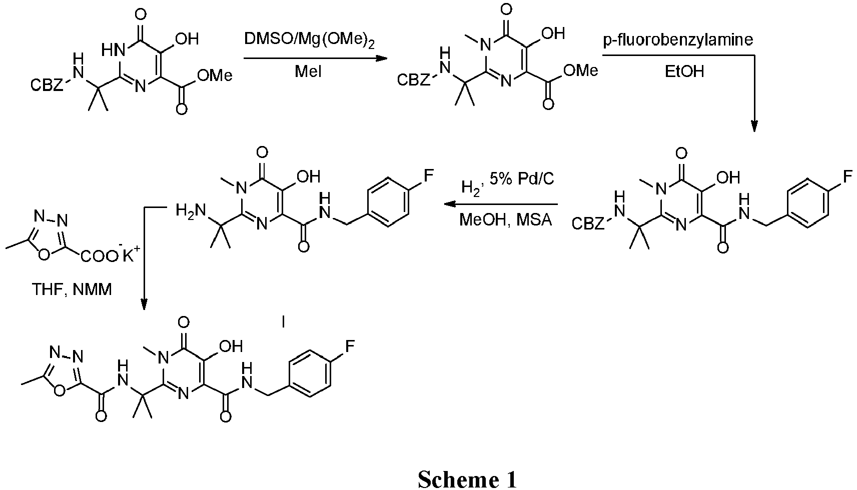

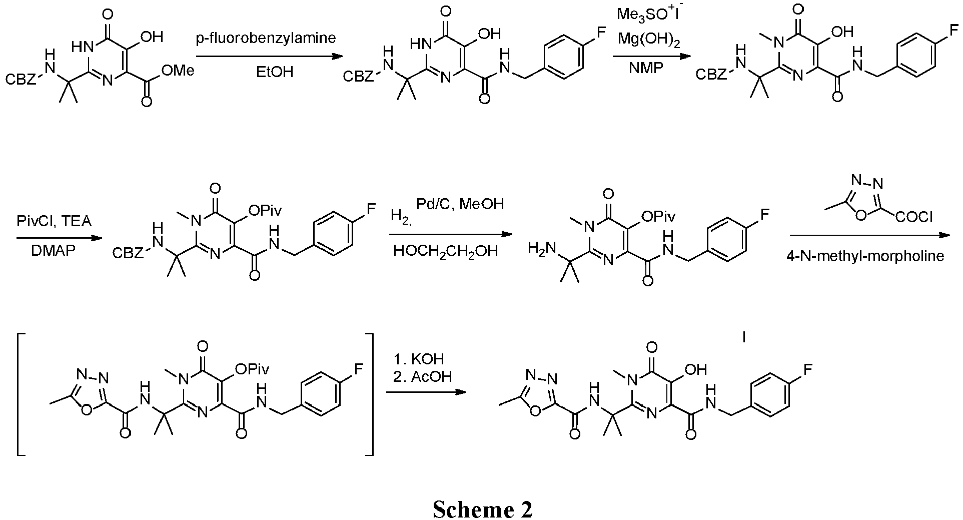

WO2009088729 discloses another method for the preparation of Raltegravir, shown above (Scheme 2).

According to the disclosure of this patent application, the methylation method of WO2006060730 is accompanied by the loss of CBZ group, the demethylation of the ester and the formation of O-methylated by-products, which affects the yield of the desired N-methyl product. Allegedly, the process disclosed in WO'729 does not include those drawbacks. However the process employs the use of N-methylpyrrolidone as a solvent and trimethylsulfoxonium iodide as the methylating agent. Additionally, it performs the reaction in high temperatures (about 100 °C) for more than 6 hours. The solvent is far from being desirable for industrial purposes and the use of methylating reagents always requires careful handling and disposal because of their very nature. Prolonged reaction time in highly elevated temperatures is also not desirable, because it renders industrial processes more energy-costing and thus less attractive from an economic aspect.

Therefore there exists a need for the provision of an improved process for the preparation of Raltegravir and key intermediates thereof via an effective methylation process, which can be employed in a chemoselective manner, uses less dangerous reagents and solvents and can be applied in an industrial scale with low cost.

### SUMMARY OF THE INVENTION

The present invention discloses a novel processes for preparing compounds useful as intermediates in the synthesis of Raltegravir.

There is disclosed a process for the preparation of compounds of formulae **II** and **IV**, wherein a methyl group is introduced selectively on the -NH- moiety of the heterocyclic ring, by a method which comprises: i) reaction with a halomethylhalosilane reagent **S** and ii) reaction with a fluoride ion source, optionally comprising further steps between them.

There is further provided a process for the preparation of Raltegravir (**I**) wherein a methyl group is introduced selectively on the -NH- moiety of the heterocyclic ring, according to the previous method.

### DEFINITIONS

The following terms shall have, for the purposes of this application, including the claims appended hereto, the respective meanings set forth below. It should be understood that when reference herein is made to a general term, such as acid, base, oxidizing agent, etc. one skilled in the field may make appropriate selections for such reagents from those given in the definitions below, as well as from additional reagents recited in the specification that follows, or from those found in literature references in the field.

An "alkyl" group refers to any saturated aliphatic hydrocarbon, including straight-chain, and branched-chain. In one embodiment, the alkyl group has 1-12 carbons designated here as C₁-C₁₂-alkyl. In another embodiment, the alkyl group has 1-6 carbons designated here as C₁-C₆-alkyl. In another embodiment, the alkyl group has 1-4 carbons designated here as C₁-C₄ alkyl. The alkyl group may be unsubstituted or substituted by one or more groups selected from halogen, hydroxyl, alkoxy carbonyl, amido, alkylamido, dialkylamido, nitro, amino, alkylamino, dialkylamino, carboxyl, thio and thioalkyl. Each possibility represents a separate embodiment of the present invention.

An "aryl" group refers to an aromatic ring system containing from 6-14 ring carbon atoms. The aryl ring can be a monocyclic, bicyclic, tricyclic and the like. Non-limiting examples of aryl groups are phenyl, naphthyl including 1-naphthyl and 2-naphthyl, and the like. The aryl group can be unsubstituted or substituted through available carbon atoms with one or more groups defined hereinabove for alkyl. Each possibility represents a separate embodiment of the present invention.

An "alkylaryl" group is an alkyl group as defined herein bonded to an aryl group as defined herein. The aryl group can be unsubstituted or substituted through available carbon atoms with one or more groups defined hereinabove for alkyl.

"Halide" or "halo" refers to F, Cl, Br, or I.

The term "hydroxyl protecting groups" refers to protecting groups suitable for the protection of the hydroxyl moiety, which may also be referred to as "protected hydroxyl group". Such groups are known and exemplified in the art, such as in Greene's Protective Groups on Organic Synthesis 4th Edition, John Wiley & Son, Peter G. M. Wuts, Theodora W. Greene, Print ISBN: 9780471697541. Preferred protecting groups are trityl, benzyl, naphthyl, p-methoxybenzyl, p-nitrobenzyl, benzoyl, a substituted benzoyl, acetyl, a substituted acetyl, pivaloyl, trimethylsilyl, triethylsilyl, isopropyldimethylsilyl, tert-butyldimethylsilyl, tert-butyldiphenylsilyl, thexyldimethylsilyl, allyl, methoxymethyl, (2-methoxyethoxy)methyl, tetrahydropyranyl.

"Silyl protecting group" is a protecting group wherein the oxygen is linked with a silicon atom. Silyl protecting groups are disclosed in the textbook mentioned above. Preferred silyl protecting groups are trimethylsilyl, triethylsilyl, isopropyldimethylsilyl, tert-butyldimethylsilyl, tert-butyldiphenylsilyl, thexyldimethylsilyl.

A "silylating agent" may be any reagent which is able to insert a silyl protecting group to a free hydroxyl. Silyl protecting groups are defined above.

The term "amine protecting group" refers to protecting groups suitable for the protection of the amine moiety, which may also be referred to as "protected amine group". Such groups are known and exemplified in the art, such as in Greene's Protective Groups on Organic Synthesis 4th Edition, John Wiley & Son, Peter G. M. Wuts, Theodora W. Greene, Print ISBN: 9780471697541. Preferred amine protecting groups are carbamates, amides, N-alkyl, N-aryl, N-silyl amino and N-sulfonyl derivatives. More preferred amine protecting groups are tert-butoxycarbonyl (Boc), benzyloxycarbonyl (Cbz), allyloxycarbonyl (Alloc), ethyloxycarbonyl, 9-fluorenylmethoxycarbonyl (Fmoc), 2,2,2-trichloroethyl carbonate (Troc), formyl, acetyl, trifluoroacetyl, benzoyl (Bz), benzyl (Bn), allyl (All), trityl (Tr), trimethoxyphenylmethylene, trimethylsilyl (TMS), tert-butyldimethylsilyl, (TBS), p-methoxybenzyl (PMB), p-halo-benzyl, diphenylmethyl, naphthylmethyl, benzenesulfonate, tosylate.

"Base" when used herein includes hydroxides or alkoxides, hydrides, or compounds such as amine and its derivatives, that accept protons in water or solvent. Thus, exemplary bases include, but are not limited to, alkali or alkaline earth metal hydroxides and alkoxides (i.e., MOR, wherein M is an alkali metal such as potassium, lithium, or sodium, and R is hydrogen or alkyl, as defined above, more preferably where R is straight or branched chain C1-5 alkyl, thus including, without limitation, potassium hydroxide, potassium tert-butoxide, potassium tert-pentoxide, sodium hydroxide, sodium, potassium or magnesium methoxide, sodium tert-butoxide, lithium hydroxide, magnesium hydroxide, calcium hydroxide, barium hydroxide); alkali metal hydrides (i.e., MH, wherein M is as defined above, thus including, without limitation, sodium, potassium, and lithium hydrides); alkylated disilazanes, such as, for example, potassium hexamethyldisilazane and lithium hexamethyldisilazane; carbonates such as potassium carbonate (K₂CO₃), sodium carbonate (Na₂CO₃), potassium bicarbonate (KHCO₃), and sodium bicarbonate (NaHCO₃), alkyl ammonium hydroxides such as tetrabutyl ammonium hydroxide (TBAH) and so forth. The term "base" may also refer amines and their derivatives, for example, trialkyl amine (e.g., Et₃N, diisopropylethyl amine, etc.), aromatic amine (e.g., Ph-NH₂, PhN(Me)H, etc.) or other heterocyclic amines (morpholine, N-methyl-morpholine, etc); alkali metal alkoxides; alkali metal hydrides; alkylated disilazides; and non-aqueous carbonates.

The term "acid" might refer to the following: strong acids such as mineral acids, e. g. sulphuric acid, phosphoric acid, boric acid, nitric acid or hydrohalic acids; strong organic carboxylic acids, such as alkanecarboxylic acids of 1 to 4 carbon atoms which are unsubstituted or substituted (e. g., by halogen), such as acetic acid and trifluoroacetic acid; saturated or unsaturated dicarboxylic acids, for example oxalic, malonic, succinic, maleic, fumaric, phthalic or tetraphthalic; hydroxycarboxylic acids, for example ascorbic, glycolic, lactic, malic, tartaric or citric acid; aminoacids, for example aspartic or glutamic acid; benzoic acid; organic sulfonic acids, such as (C1-4)-alkyl- or aryl-sulfonic acids which are substituted or unsubstituted (for example, by a halogen) such as methane-, trifluoromethane or p-toluene sulfonic acid.

The term "acid" might further refer to heteropolyacids. Heteropolyacids are the acidic forms of polyoxometalates. They usually contain a metal, such as tungsten, molybdenum or vanadium, oxygen, a hetero atom (an element of the p-block of the periodic table, such as silicon phosphorus or arsenic) and acidic hydrogen atoms. A more comprehensive definition is provided in "Enviromentally Bening Catalysts: For Clean Organic Reactions" , Part 3 (Transition Metal-Substituted Salt of Tungsten-Based Polyoxometalate-Supported Mesoporous Silica as a Catalyst for Organic Transformation Reactions, Chapter I , Springer Publications, ISBN 978-94-007-6709-6. Examples of heteropolyacids are phosphotungstic acid (H₃PW₁₂O₄₀), phoshpomolybdic acid (H₃PMo₁₂O₄₀) and silicotungstic acid (H₄SiW₁₂O₄₀), silicomolybdic acid (H₄SiMo₁₂O₄₀).

Acceptable salts of the compounds prepared herein include suitable acid addition salts thereof. Salts are formed, for example, with strong acids such as mineral acids, e. g. sulphuric acid, phosphoric acid, nitric acid, boric acid or hydrohalic acids; with strong organic carboxylic acids, such as alkanecarboxylic acids of 1 to 4 carbon atoms which are unsubstituted or substituted (e. g., by halogen), such as acetic acid and trifluoroacetic acid; with saturated or unsaturated dicarboxylic acids, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as (C1-4)-alkyl- or aryl-sulfonic acids which are substituted or unsubstituted (for example, by a halogen) such as methane- or p-toluene sulfonic acid.

Pharmaceutically acceptable salts are salts that retain the desired biological activity of the parent compound and do not impart undesired toxicological effects. Examples of such salts are (a) acid addition salts formed with inorganic acids, for example hydrochloric acid, hydrobromic acid, sulfuric acid, phosphoric acid, nitric acid and the like; and salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, palmitic acid, alginic acid, polyglutamic acid, naphthalenesulfonic acid, methanesulfonic acid, p-toluenesulfonic acid, naphthalenedisul fonic acid, polygalacturonic acid, and the like; (b) salts formed from elemental anions such as chlorine, bromine, and iodine, and (c) salts derived from bases, such as ammonium salts, alkali metal salts such as those of sodium and potassium, alkaline earth metal salts such as those of calcium and magnesium, and salts with organic bases such as dicyclohexylamine and N-methyl-D-glucamine. A review of suitable pharmaceutical salts may be found in Berge et al., J. Pharm. Sci., 66, 1, 19 (1977).

Additionally, it should be understood in the methods of preparation and claims herein, that the pronoun "a", when used to refer to a reagent, such as "a base", "a solvent" and so forth, is intended to mean "at least one" and thus, include, where suitable, single reagents as well as mixtures of reagents.

### DETAILED DESCRIPTION OF THE INVENTION

The present invention discloses novel methods for the production of intermediates used in the preparation of Raltegravir.

According to a first embodiment of the present invention, there is provided a process for the preparation of compound of formula **II**, 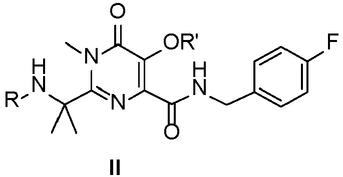 wherein R represents hydrogen or an amine protecting group and R' represents hydrogen or a hydroxyl protecting group, comprising:
a) treating compound of formula **III**, wherein R" represents hydrogen, alkyl, aryl or aralkyl group, with a halomethylhalosilane reagent **S**, of general formula HalCH₂Si(Hal)A₂, wherein each Hal, independent of one another, represents a halogen atom and A represents alkyl, aryl or aralkyl; 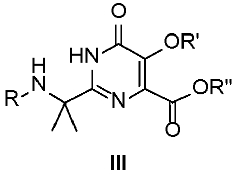
b) treating the resulting intermediate product **1** with p-fluorobenzylamine;
c) treating the resulting intermediate product **2** with a fluoride ion source to provide compound of formula **II.**

Step a comprises the formation of intermediate product **1,** upon reaction of compound of formula **III** with a halomethylhalosilane reagent (**S**)**.** It has been found that the use of such reagents, instead of more conventional methylating agents used in prior art, results in the selective preparation of compound of formula **II**, against O-methylated isomers, with high overall yield.

In a preferred embodiment both halogens of the **S** reagent are chloro atoms. In a more preferred embodiment reagent **S** is (chloromethyl)dimethylchlorosilane ClCH₂SiMe₂Cl (**S-1**).

Typically 1.0-3.0 equivalents of the **S** reagent are used. Preferable are 1.0-2.0 equivalents. More preferable are 1.0-1.5 equivalents.

The reaction may also comprise the use of an activating agent. This agent activates the nitrogen atom of the - NH - moiety of the heterocyclic ring, to facilitate the reaction with the **S** reagent. Preferable activating agents are silylating agents. Preferable silylating agents are trialkylsilyl halides, bis(trimethylsilyl)acetamide, tetrachlorosilane, hexamethyldisilazane, diphenylmethylchlorosilane, t-butyldimethylchlorosilane. Even more preferable is hexamethyldisilazane. More preferable are trimethylsilylchloride, hexamethyldisilazane.

The reaction is typically performed in polar organic solvents. Preferable are aprotic polar organic solvents.

The temperature of the reaction may vary between room temperature and 200 °C. Preferable is a range between room temperature and 100 °C. More preferable is a range between room temperature and 80 °C.

Step b comprises the formation of the amide bond with p-fluorobenzylamine, to provide intermediate product **2.** The reaction may be performed with 1.0-3.0 equivalents of p-fluorobenzylamine. In a preferred embodiment the equivalents used are 1.0-1.5. In a more preferred embodiment the equivalents used are 1.2-1.3.

The reaction may be performed in a polar organic solvent. Preferable are polar protic organic solvents. More preferable are alcohols.

The temperature may vary between room temperature and 200 °C. Preferable is a range between room temperature and 150 °C.

The reaction is performed preferably in the presence of a base. The base may be an alkali metal or alkaline earth hydroxide or alkoxide, carbonates, alkyl ammonium hydroxides, amines and their derivatives. Preferable are amines and their derivatives.

Step c comprises the cleavage of a silyl moiety from intermediate product **2**, thereby providing compound **II.** Reagents suitable for this conversion are those which can behave as fluoride ion sources. Preferable are fluoride salts of metals or of tertiary amines. More preferable are fluoride salts of alkali metals and of tertiary amines. Even more preferable is potassium fluoride.

The reaction may be performed in a polar organic solvent. More preferable are alcohlols, ethers and esters. Even more preferable are methanol, ethanol, ethyl acetate and tetrahydrofuran.

In preferred embodiments, any of steps a, b, c or all of the steps a, b, c may be performed without isolation of intermediates. In a more preferred embodiment steps a, b, c are all performed without isolation of intermediates. This is beneficial, particularly for industrial purposes, because the elimination of isolation of intermediates contributes to a higher overall yield and at the same time provides for a faster and simpler process.

According to a second embodiment of the present invention, there is provided a process for the preparation of Raltegravir or a pharmaceutically acceptable salt thereof, comprising steps a-c as described above and further comprising converting compound of formula **II** to Raltegravir or a pharmaceutically acceptable salt thereof. The conversion of compound **II** to Raltegravir is disclosed in prior art. By way of example, compound of formula **II** can be converted to Raltegravir by the process disclosed in WO2006060730 or WO2009088729.

According to a third embodiment of the present invention, there is provided a process for the preparation of compound of formula **IV**, 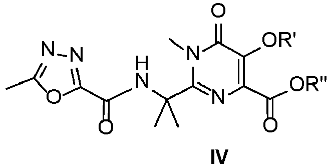 wherein R' and R" are defined as above, comprising:
a) treating compound of formula **III**, as defined above, with a halomethylhalosilane reagent **S,** as defined above;
d) when R is other than hydrogen, removing protecting group R from resulting intermediate product **1**;
e) treating resulting intermediate product **3** with compound of formula **V**, wherein Y represents halogen atom, or hydroxyl group, or alkoxide moiety ― O-X+, X being an alkali metal or alkaline earth metal; 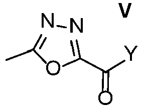
f) treating resulting intermediate product **4** with a fluoride ion source to provide compound of formula **IV**.

Step a may be performed as described above.

Step d is a deprotection reaction which provides intermediate product **3** and may be performed in accordance with the nature of the protecting group. Standard deprotection reagents and conditions are illustrated in Greene's Protective Groups on Organic Synthesis 4th Edition, John Wiley & Son, Peter G. M. Wuts, Theodora W. Greene, Print ISBN: 9780471697541.

Step e comprises the formation of the amide bond with the oxadiazole moiety, to provide intermediate product **4.** The reaction may be performed with 1.0-3.0 equivalents of compound of formula **V.**

The reaction may be performed in a polar organic solvent. Preferable are polar aprotic organic solvents.

The temperature may vary between room temperature and 200 °C. Preferable is a range between room temperature and 150 °C.

The reaction is performed preferably in the presence of a base. The base may be an an alkali metal or alkaline earth hydroxide or alkoxide, carbonates, alkyl ammonium hydroxides, amines and their derivatives. Preferable are amines and their derivatives.

Step f comprises the cleavage of a silyl moiety from intermediate product **4**, thereby providing compound **IV.** It may be performed as disclosed for step c.

In preferred embodiments, any of steps a, d,e,f or all of the steps a, d, e, f may be performed without isolation of intermediates. In a more preferred embodiment steps a, d, e, f are all performed without isolation of intermediates. As disclosed above, this is beneficial.

According to a fourth embodiment of the present invention, there is provided a process for the preparation of Raltegravir or a pharmaceutically acceptable salt thereof, comprising steps a and d-f as described above and further comprising converting compound of formula **IV** to Raltegravir. The conversion of compound **IV** to Raltegravir is disclosed in prior art. By way of example, compound of formula **IV** can be converted to Raltegravir by the method disclosed in WO2013098854.

According to a fifth embodiment of the present invention, there is provided a process for the preparation of compounds of formulae **II**, **IV**, or salts thereof, wherein a methyl group is introduced selectively on the -NH- moiety of the heterocyclic ring, by a method which comprises: 1) reaction with a halomethylhalosilane reagent **S**, as defined above and 2) reaction with a fluoride ion source, optionally comprising further steps between them. According to this embodiment, step 1 comprises a reaction with the halomethylhalosilane reagent **S**, which is defined as above. In a preferred embodiment both halogens of the S reagent are chloro atoms. In a more preferred embodiment reagent **S** is ClCH₂SiMe₂Cl (**S-1**).

The reaction may also comprise the use of an activating agent. This agent activates the nitrogen atom of the - NH - moiety of the heterocyclic ring, to facilitate the reaction with the S reagent. Preferable activating agents are silylating agents. Preferable silylating agents are trialkylsilyl halides, bis(trimethylsilyl)acetamide, tetrachlorosilane, hexamethyldisilazane, diphenylmethylchlorosilane, t-butyldimethylchlorosilane. Even more preferable is hexamethyldisilazane. More preferable are trimethylsilylchloride, hexamethyldisilazane.

The reaction of step 2 comprises the cleavage of a silyl moiety. Reagents suitable for this conversion are those which can behave as fluoride ion sources. Preferable are fluoride salts of metals or of tertiary amines. More preferable are fluoride salts of alkali metals and of tertiary amines. Even more preferable is potassium fluoride.

The reaction may be performed in a polar organic solvent. More preferable are alcohlols, ethers and esters. Even more preferable are methanol, ethanol, ethyl acetate and tetrahydrofuran.

Advantageously, this method provides selective methylation on the -NH- moiety of the heterocyclic ring against the neighboring oxygen atom. The method may be performed irrespectively of any further steps interposed between steps 1 and 2. Hence the present method may be performed with or without further steps between steps 1 and 2.

According to a sixth embodiment of the present invention, there is provided a process for the preparation of Raltegravir or pharmaceutically acceptable salts thereof, wherein a methyl group is introduced selectively on the -NH- moiety of the heterocyclic ring, by a method which comprises: 1) reaction with a halomethylhalosilane reagent **S** and 2) reaction with a fluoride ion source, optionally comprising further steps between them. Reactions of steps 1 and 2 may be performed as described above. The method may be performed irrespectively of any further steps interposed between steps 1 and 2. Hence the present method may be performed with or without further steps between steps 1 and 2.

According to a seventh embodiment of the present invention, there is provided a process for the preparation of compound of formula **VI**, wherein R, R' and R" are defined as above, comprising: a) treating compound of formula **III**, wherein R, R' and R" are defined as above, with a halomethylhalosilane reagent **S**, of general formula HalCH₂Si(Hal)A₂, wherein Hal and A are defined as above and g) treating the resulting intermediate product **1** with a fluoride ion source to provide compound of formula **VI.**

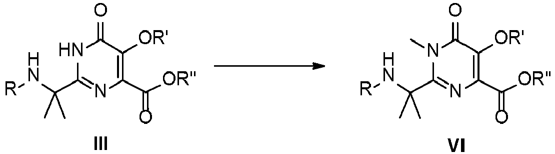

Step a) may be performed as described above. Step g) may be performed in accordance with step c).

According to an eighth embodiment of the present invention, there is provided the use of a halomethylhalosilane reagent **S** for the preparation of compounds of formulae **II, IV, VI** or salts thereof, or Raltegravir or pharmaceutically acceptable salts thereof, through a method of selective introduction of a methyl group on the -NH- moiety of the heterocyclic ring. The halomethylhalosilane reagent **S** may be used as set forth above.

Within the scope of the present invention, each of the intermediate products **1-4** may comprise a single compound or mixtures of compounds. The chemical profile of those intermediate products depends on the choice of reagent **S**, the exact structure of the compounds used as starting materials, compounds of formulae **III** and **IV**, and the reaction conditions of steps a-f. These factors may influence the multifunctional character of reagent **S**, resulting in more or less complex chemical structures. This, however, does not jeopardize the selective character of the methylation method of the present invention.

In a preferred embodiment, intermediate product **1** comprises a compound of formula **1_{A}**, wherein R, R' and R" are as defined above. In a more preferred embodiment, intermediate product **1** is represented by compound of formula **1_{A}**.

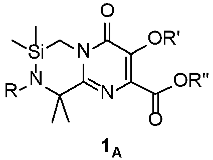

In a preferred embodiment, intermediate product 1 comprises a compound of formula **1_{B}**, wherein R, R' and R" are as defined above. In a more preferred embodiment, intermediate product **1** is represented by compound of formula **1_{B}**.

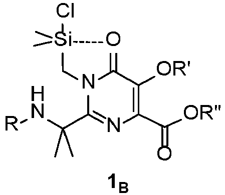

In a preferred embodiment, intermediate product **2** comprises a compound of formula **2_{A}**, wherein R and R' are as defined above. In a more preferred embodiment, intermediate product **2** is represented by compound of formula **2_{A}**.

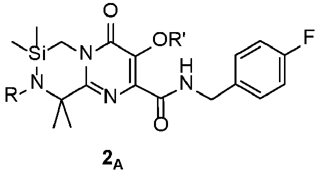

In a preferred embodiment, intermediate product **2** comprises a compound of formula **2_{B}**, wherein R and R' are as defined above. In a more preferred embodiment, intermediate product **2** is represented by compound of formula **2_{B}**.

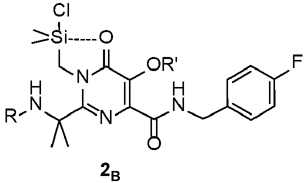

In a preferred embodiment, intermediate product **3** comprises a compound of formula **3_{A}**, wherein R' and R" are as defined above. In a more preferred embodiment, intermediate product **3** is represented by compound of formula **3_{A}**.

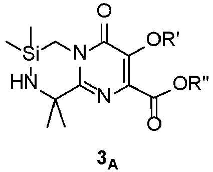

In a preferred embodiment, intermediate product **3** comprises a compound of formula **3_{B}**, wherein R' and R" are as defined above. In a more preferred embodiment, intermediate product 3 is represented by compound of formula **3_{B}**.

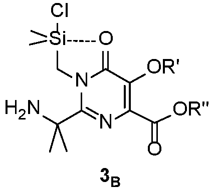

In a preferred embodiment, intermediate product **4** comprises a compound of formula **4_{A}**, wherein R' and R" are as defined above. In a more preferred embodiment, intermediate product **4** is represented by compound of formula **4_{A}**.

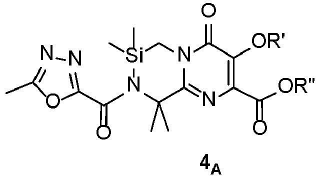

In a preferred embodiment, intermediate product **4** comprises a compound of formula **4_{B}**, wherein R' and R" are as defined above. In a more preferred embodiment, intermediate product **4** is represented by compound of formula **4_{B}**.

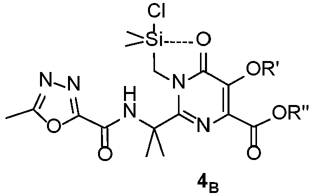

According to a ninth embodiment of the present invention, there is provided a process for the preparation of intermediate product **1**, comprising:
a) treating compound of formula **III**, as defined above, with a halomethylsilyl reagent **S**, as defined above;
g) isolating intermediate product **1.**

According to a tenth embodiment of the present invention, there is provided intermediate product **1**, obtainable by a process as described above.

According to a eleventh embodiment of the present invention, there are provided compounds of formulae **1_{A}** and **1_{B}**.

According to an twelfth embodiment of the present invention, there is provided a process for the preparation of intermediate product **2**, comprising:
a) treating compound of formula **III**, as defined above, with a halomethylsilyl reagent **S**, as defined above;
b) treating the resulting intermediate product **1** with p-fluorobenzylamine;
h) isolating intermediate product **2.**

According to a thirteenth embodiment of the present invention, there is provided intermediate product **2**, obtainable by a process as described above.

According to a fourteenth embodiment of the present invention, there are provided compounds of formulae **2_{A}** and **2_{B}**.

According to a fifteenth embodiment of the present invention, there is provided the use of intermediate products **1, 2** or compounds of formulae **1_{A}**, **1_{B}**, **2_{A}** or **2_{B}** for the preparation of compound of formula **II.**

According to a sixteenth embodiment of the present invention, there is provided a process for the preparation of intermediate product **3,** comprising:
a) treating compound of formula **III**, as defined above, with a halomethylsilyl reagent **S**, as defined above;
d) when R is other than hydrogen, removing protecting group R from resulting intermediate product **1**;
i) isolating intermediate product **3.**

According to a seventeenth embodiment of the present invention, there is provided intermediate product **3**, obtainable by a process as described above.

According to a eighteenth embodiment of the present invention, there are provided compounds of formulae **3_{A}** and **3_{B}**.

According to an nineteenth embodiment of the present invention, there is provided a process for the preparation of intermediate product **4**, comprising:
a) treating compound of formula **III**, as defined above, with a halomethylsilyl reagent **S**, as defined above;
d) when R is other than hydrogen, removing protecting group R from resulting intermediate product **1**;
e) treating resulting intermediate product **3** with compound of formula **V**, as defined above;
j) isolating intermediate product **4.**

According to a twentieth embodiment of the present invention, there is provided intermediate product **4**, obtainable by a process as described above.

According to a twenty-first embodiment of the present invention, there are provided compounds of formulae **4_{A}** and **4_{B}**.

According to a twenty-second embodiment of the present invention, there is provided the use of intermediate products **3, 4** or compounds of formulae **3_{A}**, **3_{B}**, **4_{A}** or **4_{B}** for the preparation of compound of formula **IV.**

According to a twenty-third embodiment of the present invention, there is provided the use of intermediate products **1, 2, 3** or **4** or compounds of formulae **1_{A}**, **1_{B}**, **2_{A}**, **2_{B}**, **3_{A}**, **3_{B}**, **4_{A}** or **4_{B}** for the preparation of Raltegravir or pharmaceutically acceptable salts thereof.

According to a twenty-fourth embodiment of the present invention, there is provided the use of intermediate product **1** or compounds of formulae **1_{A}**, **1_{B}** for the preparation of compound of formula **VI.**

### EXAMPLES

### EXAMPLE 1

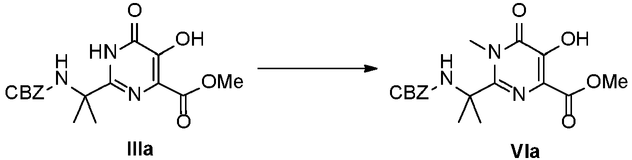

A 500 ml 3-necked round bottom flask, equipped with a reflux condenser and a thermometer is charged with 15.0 g (41.5 mmol) methyl 2-(2(((benzyloxy)carbonyl)amino)propan-2-yl)-5,6-dihydroxy-1,6dihydropyrimidine-4-carboxylate **IIIa** and 150 ml acetonitrile under inert atmosphere at 20-30 °C. To the resulting suspension is added under stirring 8.7 ml hexamethyldisilazane (41.5 mmol; 1.0 equiv) and mixture is heated up at 80 °C for 1 h. While still hot 6.0 ml (chloromethyl)-dimethylchlorosilane (45.7 mmol, 1.10 equivs) is added and heating is maintained for 2 h. Reaction progress is monitored by TLC. Upon completion the volatiles are evaporated off and the residue is further dried under high vacuum for at least 30 min. To the residue is added 75 ml ethyl acetate and mixture is heated up and stirred until a clear solution arises. 3.0 g potassium fluoride (51.9 mmol; 1.25 equivs) is added and stirring is maintained for approximately 1h. Upon completion, 50 ml demineralized water is added. After stirring for 10 min, the mixture is left to settle. The upper organic phase is collected and washed with 50 ml water, then dried over anhydrous sodium sulphate, filtered and evaporated down to afford **VIa** as off-white solid (12.1 g; 78% yield; >98% purity).

### EXAMPLE 2

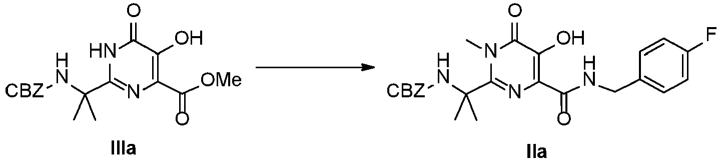

In a round-bottomed flask 1.0 g of methyl 2-(2-(benzyloxycarbonylamino)propan-2-yl)-5-hydroxy-6-oxo-1,6-dihydropyrimidine-4-carboxylate (compound of formula **IIIa**) and 16mL Acetonitrile are charged at 20-25 °C and the mixture is stirred for 5 min. To the suspension 0.58 mL Hexamethyldisilazane is added. The solution is heated to reflux temperature (80 °C) and it is stirred at that temperature for 1hour. Then, 0.4 mL of Chloro(chloromethyl)dimethylsilane is added over 5min. at reflux temperature. The mixture is stirred for another 4 hours at reflux temperature (80°C). Then the mixture is cooled to 20-25 °C and filtered under vacuum. The filtered solid is rinsed with 5mL Acetonitrile. The filtrates are evaporated to give 1.4 g of crude intermediate product **1.**

To the above mentioned product, 5mL of Methanol are added till complete dissolution. To this solution, 0.46mL of Triethylamine and 0.38mL of 4-Fluorobenzylamine are added. The solution is heated to reflux temperature (65°C) and stirred at that temperature for 2 hours. Then, the mixture is cooled to 20-25°C. The solution is concentrated to give 1.96 g of crude intermediate product **2.**

To the above mentioned product 8mL of Tetrahydrofuran and 0.5 g of Cesium Fluoride are added and the mixture is heated to reflux temperature (66°C). The mixture is stirred at that temperature for 45min. and then cooled to 20-25°C. Another 10mL Tetrahydrofuran is added and the mixture is stirred at 20-25°C for 30min. The mixture is filtered under vacuum and the solid is rinsed with 5mL Tetrahydrofuran and suck-dried to give 1.38g off-white solid (compound of formula **IIa**).

### EXAMPLE 3

A 2 L 3-necked round bottom flask, equipped with a mechanical stirrer, a reflux condenser and a thermometer is charged with 150.0 g (0.42 mol) methyl 2-(2(((benzyloxy)carbonyl)amino)propan-2-yl)-5,6-dihydroxy-1,6dihydropyrimidine-4-carboxylate **IIIa** and 0.75 L acetonitrile under inert atmosphere at 20-30 °C. To the resulting suspension is added under stirring 87 ml hexamethyldisilazane (0.42 mol; 1.0 equiv) and mixture is heated up at 80 °C for 1 h. While still hot 60.4 ml (chloromethyl)-dimethylchlorosilane is added over 10-15 min and heating is maintained for 2 h. Reaction progress is monitored by TLC. Upon completion the reaction is evaporated down and the residue is further dried under high vacuum for at least 30 min. To the residue is added 0.75 L methanol and mixture is stirred until a clear solution arises. 69.7 ml (0.50 mol; 1.20 equivs) triethylamine is added followed by 57.2 ml (0.50 mol; 1.20 equivs) 4-fluoro-benzylamine and the resulting mixture is heated up to 65 °C for 1 h. While still hot potassium fluoride (0.53 mol; 1.25 equivs) is added in one portion and heating maintained for 1-2 h. Upon completion of the desilylation, a colorless solid is separated from the reaction mixture. Reaction is cooled down to ambient temperature and 1.12 L demineralized water is added and slurry is stirred for 1 h. Crude Benzyl (2-(4-((4-fluorobenzyl)carbamoyl)-5-hydroxy-1-methyl-6-oxo-1,6-dihydropyrimidin-2-yl)propan-2-yl)carbamate **IIa** is afforded by filtration under vacuum. The wet cake is washed with 0.45 L methanol/water (1:2) and suck dried for 1 h. The colorless solid is collected and dried further under vacuum at 30 °C for 10 h. After recrystallization with methanol/water = 3:1, pure **IIa** is obtained as colorless solid (126.8 g, 82%).

### EXAMPLE 4

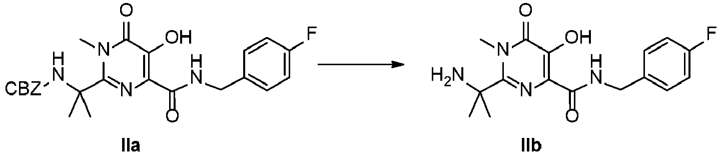

A 1L r.b. flask equipped with a magnetic bar is charged with 3.6 g (7.6 mmol) compound of formula **IIa**, followed by 60 ml methanol. Add 0.5 ml (8.4 mmol) glycolic acid in one portion under argon atmosphere. Add 0.72 g 5% Pd/C (50% w/w) and degas reaction mass under Argon atmosphere. Reaction mass is hydrogenated in atmospheric pressure for 10 h. The reaction mass is filtered and catalyst is washed with 30 ml methanol. The combined filtrate is concentrated to a total volume of 35 ml. Add 1.27 ml (9.1 mmol) triethylamine dropwise over a period of 30 min. Stir the resulting slurry at 0-5 °C for 1h. Filter solid and wash with 5 ml methanol. Solid is dried under vacuum to afford 1.95 g of compound of formula **IIb**.

### EXAMPLE 5

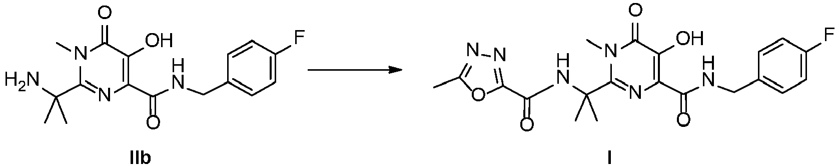

A r.b. flask (flask A) is charged with 5.0 g (14.95 mmol) of compound of formula **IIb** followed by 40 ml dichloromethane. To the resulting suspension is added 11.6 ml trimethylsilyl chloride (91.64 mmol, 6.13 eq.) and the mixture is heated up to 45 °C for 1h. The reaction mass is cooled down to -10 °C and 4.8 ml N-methylmorpholine (43.8 mmol, 2.93 eq.) is added dropwise over 10 minutes and the resulting mixture is stirred further for 1h at the same temperature. Another r.b. flask (flask B) is charged with 3.73 g (22.43 mmol, 1.50 eq.) potassium 5-methyl-1,3,4-oxadiazole-2-carboxylate **Va** (potassium salt of compound of formula **V**) followed by 40 ml dichloromethane and mixture is cooled down to -10 °C. 0.06 ml dimethylformamide (0.75 mmol, 0.05 eq.) is added followed by 1.93 ml oxalyl chloride (22.43 mmol, 1.50 eq.). Stirring is maintained for 45-60 minutes before the slurry of flask A is transferred to flask B over 15 minutes at -10 °C. The resulting mixture is stirred further for 1h. 100 ml demineralized water is added slowly to the reaction mass and the latter is left to reach ambient temperature and stirred there for 15 minutes. The resulting mixture is filtered through a celite bed and the wet cake is washed with 20 ml dichloromethane. The filtrate is left to settle and the organic phase is collected and evaporated down under vacuum at 40 °C. 5.16 g of compound of formula I is obtained.

## Claims

1. A process for the preparation of compound of formula **II,** wherein R represents hydrogen or an amine protecting group and R' represents hydrogen or a hydroxyl protecting group, comprising: 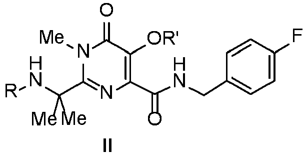
a) treating compound of formula **III**, wherein **R**" represents hydrogen, alkyl, aryl or aralkyl group, with a halomethylhalosilane reagent **S**, of general formula HalCH₂Si(Hal)A₂, wherein each Hal, independent of one another, represents a halogen atom and A represents alkyl, aryl or aralkyl; 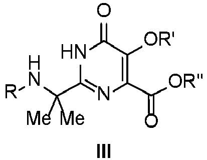
b) treating the resulting intermediate product 1 with p-fluorobenzylamine, wherein product 1 is represented by compound of formula 1_{A} or compound of formula 1_{B}; 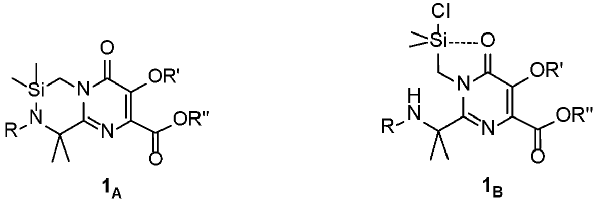
c) treating the resulting intermediate product 2 with a fluoride ion source to provide compound of formula **II,** wherein product 2 is represented by compound of formula 2_{A} or compound of formula 2_{B}.

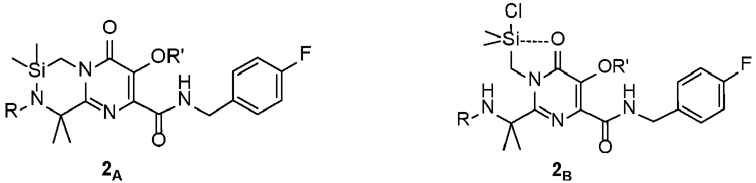

2. A process for the preparation of Raltegravir or a pharmaceutically acceptable salt thereof, comprising steps a-c as described in claim 1 and further comprising converting compound of formula **II** to Raltegravir or a pharmaceutically acceptable salt thereof.

3. A process for the preparation of compound of formula IV, wherein R' and R" are as defined in claim 1, comprising: 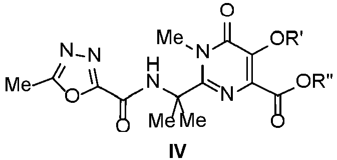
a) treating compound of formula **III**, as defined in claim 1, with a halomethylhalosilane reagent **S**, as defined in claim 1;
d) when R is other than hydrogen, removing protecting group R from resulting intermediate product **1**, as defined in claim 1;
e) treating resulting intermediate product 3 with a compound of formula V, wherein Y represents halogen atom, or hydroxyl group, or alkoxide moiety ― O⁻X⁺, X being an alkali metal or alkaline earth metal; 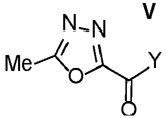 wherein product 3 is represented by compound of formula 3_{A} or compound of formula 3_{B} 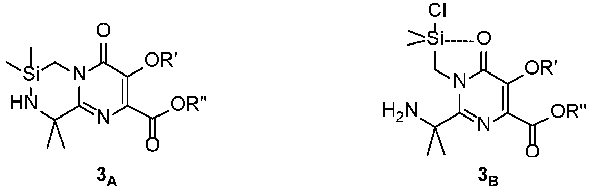
f) treating resulting intermediate product **4** with a fluoride ion source to provide compound of formula **IV**, wherein product 4 is represented by compound of formula 4a or compound of formula 4_{B}

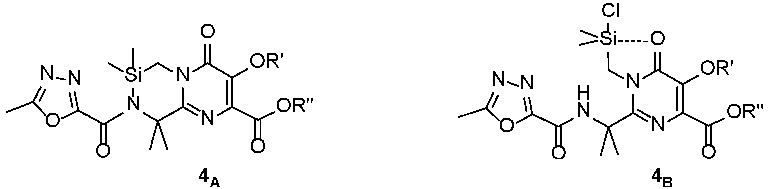

4. A process for the preparation of Raltegravir or a pharmaceutically acceptable salt thereof, comprising steps a and d-f as described in claim 3 and further comprising converting compound of formula **IV** to Raltegravir or a pharmaceutically acceptable salt thereof.

5. A process for the preparation of compound of formula **VI**, 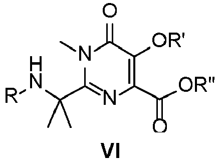 wherein R, R' and R" are defined in claim 1, comprising: a) treating compound of formula **III**, wherein R, R' and R" are defined as in claim 1, with a halomethylhalosilane reagent S, of general formula HalCH₂Si(Hal)A₂, wherein Hal and A are defined as in claim 1 and g) treating the resulting intermediate product**1**, as defined in claim 1, with a fluoride ion source to provide compound of formula **VI.**

6. A process for the preparation of compounds of formulae **II**, **IV,** as defined in claims 1 and 3, or salts thereof, wherein a methyl group is introduced selectively on the -NH- moiety of the heterocyclic ring, by a method which comprises: i) reaction with a halomethylhalosilane reagent **S**, as defined in claim 1 and ii) reaction with a fluoride ion source, optionally comprising further steps between them.

7. A process for the preparation of Raltegravir or pharmaceutically acceptable salts thereof, wherein a methyl group is introduced selectively on the -NH- moiety of the heterocyclic ring, by a method which comprises: i) reaction with a halomethylhalosilane reagent **S**, as defined in claim 1 and ii) reaction with a fluoride ion source, optionally comprising further steps between them.

8. Use of a halomethylhalosilane reagent S for the preparation of compounds of formulae **II, IV, VI** or salts thereof or Raltegravir or pharmaceutically acceptable salts thereof, through a method of selective introduction of a methyl group on the - NH - moiety of the heterocyclic ring, wherein reagent S, and compounds II, IV and VI are as defined in the previous claims.

## Patentansprüche

1. Verfahren für die Zubereitung einer Verbindung der Formel **II,** worin R Wasserstoff bzw. eine Schutzgruppe für Amine ist und R' Wasserstoff bzw. eine Hydroxyl-Schutzgruppe ist, wobei das Verfahren Folgendes umfasst: 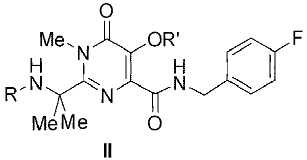
a. Behandeln der Verbindung der Formel **III**, worin R" Wasserstoff, Alkyl-, Aryl- oder Aralkyl-Gruppe ist, mit einem Halogenmethylhalogensilan-Reagenz **S** der allgemeinen Formel HalCH₂Si(Hal)A₂, worin jedes Halogen, unabhängig voneinander, ein Halogenatom ist und A Alkyl, Aryl oder Aralkyl ist; 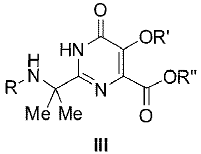
b. Behandeln des entstehenden Zwischenprodukts **1** mit p-Fluorbenzylamin, worin Produkt 1 durch die Verbindung der Formel 1_{A} oder die Verbindung der Formel 1_{B} repräsentiert wird; 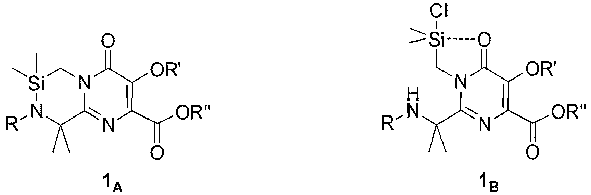
c. Behandeln des entstehenden Zwischenprodukts **2** mit einer Fluoridionenquelle, um die Verbindung der Formel **II** herzustellen, worin das Produkt 2 durch die Verbindung der Formel 2_{A} oder die Verbindung der Formel 2_{B} repräsentiert wird.

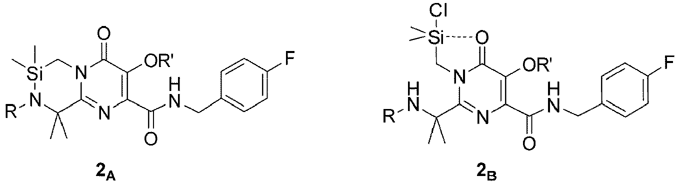

2. Verfahren für die Zubereitung von Raltegravir oder eines pharmazeutisch verwendbaren Salzes davon, welches die Schritte a-c des Anspruchs 1 umfasst und darüber hinaus das Umwandeln der Verbindung der Formel **II** in Raltegravir oder in einem pharmazeutisch verwendbaren Salz davon umfasst.

3. Verfahren für die Zubereitung der Verbindung der Formel IV, worin R' und R" so sind, wie im Anspruch 1 beschrieben, welches Folgendes umfasst: 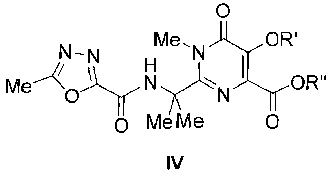
a. Behandeln der Verbindung der Formel **III**, wie diese im Anspruch 1 beschrieben wurde, mit einem Halogenmethylhalogensilan-Reagenz **S,** wie im Anspruch 1 bestimmt;
d. worin R anderes als Wasserstoff ist, indem die Schutzgruppe R vom entstehenden Zwischenprodukt 1 entfernt wird, wie im Anspruch 1 bestimmt;
e. Behandeln des entstehenden Zwischenprodukts 3 mit einer Verbindung der Formel V, worin Y ein Halogenatom oder eine Hydroxylgruppe oder ein Alkoxidanteil - O-X+ ist, wobei X ein Alkalimetall bzw. ein Alkalierdmetall ist, 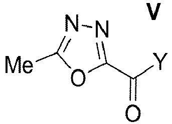 worin Produkt 3 durch die Verbindung der Formel 3_{A} bzw. die Verbindung der Formel 3_{B} repräsentiert wird. 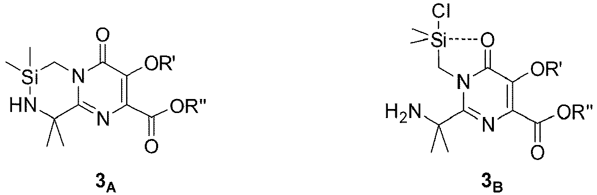
f. Behandeln des entstehenden Zwischenprodukts **4** mit einer Fluoridionenquelle, um die Verbindung der Formel **IV** herzustellen, worin das Produkt 4 durch die Verbindung der Formel 4_{A} oder die Verbindung der Formel 4_{B} repräsentiert wird.

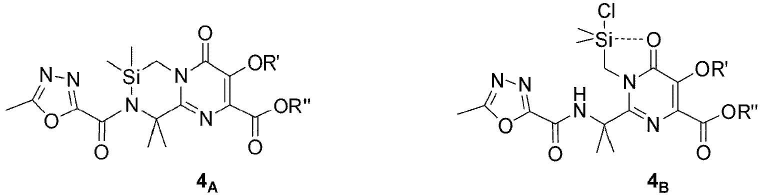

4. Verfahren für die Zubereitung von Raltegravir oder eines pharmazeutisch verwendbaren Salzes davon, welches die Schritte a und d-f des Anspruchs 3 umfasst und darüber hinaus das Umwandeln der Verbindung der Formel **IV** in Raltegravir oder in einen pharmazeutisch verwendbaren Salz davon umfasst.

5. Verfahren für die Vorbereitung der Verbindung der Formel **VI**, 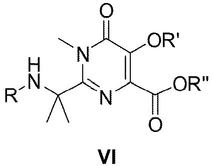 worin R, R' und R" im Anspruch 1 bestimmt werden, welches Folgendes umfasst: a) Behandeln der Verbindung der Formel **III**, worin R, R' und R" wie im Anspruch 1 bestimmt werden, mit einem Halogenmethylhalogensilan-Reagenz **S** der allgemeinen Formel HalCH2Si(Hal)A2, worin Hal und A gemäß Anspruch 1 sind; und g) Behandeln des entstehenden Zwischenprodukts 1 gemäß Anspruch 1 mit einer Fluoridionenquelle, um die Verbindung der Formel **VI** herzustellen.

6. Verfahren für die Zubereitung der Formeln **II**, **IV** gemäß den Ansprüchen 1 und 3 oder der Salze davon, worin eine Methylgruppe selektiv nach dem NH-Anteil des heterozyklischen Ringes mit einer Methode eingeführt wird, die Folgendes umfasst: i) Reaktion mit einem Halogenmethylhalogensilan-Reagenz S gemäß Anspruch 1 und ii) Reaktion mit einer Fluoridionenquelle, wahlweise mit weiteren Schritten zwischen den beiden.

7. Verfahren für die Zubereitung von Raltegravir oder von pharmazeutisch verwendbaren Salzen davon, worin eine Methylgruppe selektiv nach dem NH-Anteil des heterozyklischen Ringes mit einer Methode eingeführt wird, die Folgendes umfasst: i) Reaktion mit einem Halogenmethylhalogensilan-Reagenz S gemäß Anspruch 1 und ii) Reaktion mit einer Fluoridionenquelle, wahlweise mit weiteren Schritten zwischen den beiden

8. Verwendung von einem Halogenmethylhalogensilan-Reagenz S für die Zubereitung von einer Verbindung der Formeln II, IV, VI oder von Salzen davon oder von Raltegravir bzw. von pharmazeutisch verwendbaren Salzen davon durch eine Methode einer selektiven Einführung einer Methylgruppe nach dem NH-Anteil des heterozyklischen Ringes, worin das Reagenz S und die Verbindungen II, IV und VI in den vorigen Ansprüchen bestimmt werden.

## Revendications

1. Un procédé de préparation du composé de formule **II,** dans laquelle R représente l'hydrogène ou un groupe protecteur d'amine et R' représente l'hydrogène ou un groupe protecteur d'hydroxyle, comprenant: 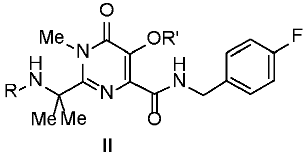
a) Un composé traitant de la formule **III** dans laquelle R" représente l'hydrogène, un groupe alkyle, aryle ou aralkyle, avec un réactif halométhylhalosilane **S,** de formule générale HalCH₂Si(Hal)A₂, dans laquelle chaque Hal, indépendamment l'un de l'autre, représente un atome d'halogène et A représente un groupe alkyle, aryle ou aralkyl; 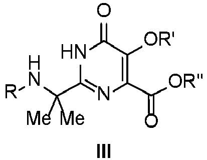
b) Le traitement du produit intermédiaire **1** qui en résulte avec p-fluorobenzylamine, dans lequel le produit 1 est représenté par le composé de formule 1_{A} ou le composé de formule 1_{B}; 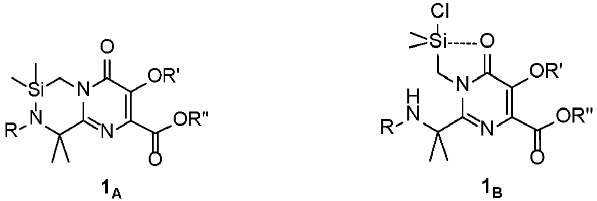
c) Le traitement du produit intermédiaire **2** qui en résulte avec source d'ions fluorure pour fournir un composé de formule **II**, dans laquelle le produit 2 est représenté par un composé de formule 2_{A} ou un composé de formule 2_{B}.

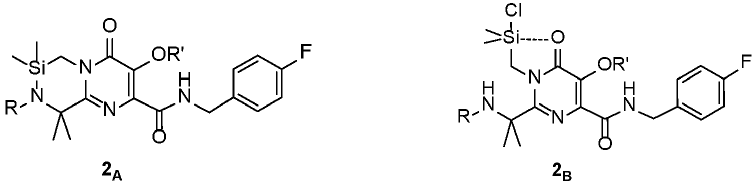

2. Un procédé de préparation du Raltégravir ou d'un sel pharmaceutiquement acceptable de celui-ci, comprenant les étapes a-c, telles que décrites dans la **1^{ère}** revendication et comprenant en outre la conversion du composé de formule **II** en Raltégravir ou un sel pharmaceutiquement acceptable de celui-ci.

3. Un procédé de préparation de composé de formule IV, dans laquelle R' et R" sont tels que définis dans la 1^{ère} revendication, comprenant: 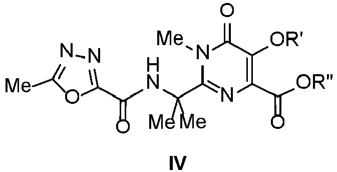
a) Un composé traitant de formule **III**, tel que défini dans la 1^{ère} revendication, avec un réactif halométhylhalosilane S, tel que défini dans la 1^{ère} revendication;
d) Lorsque R est différent de l'hydrogène, éliminer le groupe protecteur R du produit intermédiaire **1** qui en résulte, tel que défini dans la 1^{ère} revendication;
e) Un produit intermédiaire traitant **3**, qui en résulte avec un composé de formule V, dans laquelle Y représente un atome d'halogène, ou un groupe hydroxyle, ou une fraction alkoxyde- O-X+, X étant un métal alcalin ou un métal alcalino-terreux; 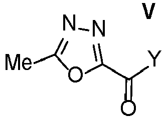 dans lequel le produit 3 est représenté par un composé de formule 3_{A} ou un composé de formule 3_{B} 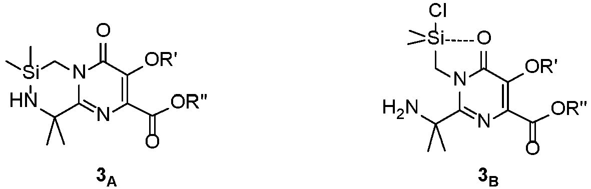
f) Un produit intermédiaire traitant **4**, qui en résulte avec résulte avec source d'ions fluorure pour fournir un composé de formule **IV**, dans laquelle le produit 4 est représenté par un composé de formule 4_{A} ou un composé de formule 4_{B}

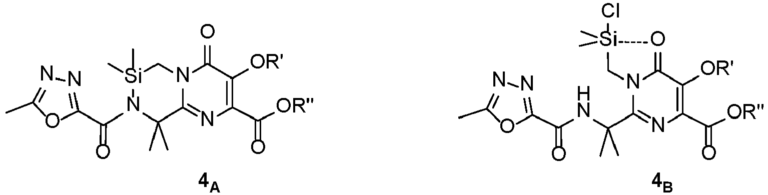

4. Un procédé de préparation du Raltegravir ou d'un sel pharmaceutiquement acceptable de celui-ci, comprenant les étapes a et d-f telles que décrites dans la 3^{ème} revendication et comprenant en outre la conversion du composé de formule **IV** en Raltégravir ou un sel pharmaceutiquement acceptable de celui-ci.

5. Un procédé de préparation de composé de formule **VI**, 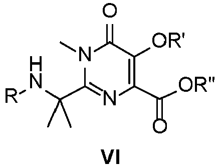 dans laquelle R' et R" sont tels que définis dans la 1^{ère} revendication, comprenant: a) Un compose traitant de formule **III**, dans laquelle R' et R" sont tels que définis dans la 1^{ère} revendication, avec un réactif halométhylhalosilane **S**, de formule générale HalCH₂Si(Hal)A₂, dans laquelle Hal et A sont définis dans la 1^{ère} revendication, et g) Un produit intermédiaire traitant 1, qui en résulte, tel que définis dans la 1^{ère} revendication, avec source d'ions fluorure pour fournir un composé de formule **II.**

6. Un procédé de préparation du composé de formules **II, IV,** tel que défini dans la 1^{ère} et 3^{ème} revendications, ou sels de celui-ci, dans lesquelles un groupe méthyl est sélectivement introduit dans le groupement des hétérocycles-NH-, par une méthode qui comprend: i) la réaction à un réactif halométhylhalosilane **S**, tel que défini dans la 1^{ère} revendication, et ii) la réaction à une source d'ions fluorure, qui comprend, facultativement, plusieurs étapes intermédiaires.

7. Un procédé de préparation du Raltégravir ou d'un sel pharmaceutiquement acceptable de celui-ci, dans laquelle un groupe méthyl est sélectivement introduit dans le groupement des hétérocycles-NH-, par une méthode qui comprend: i) la réaction à un réactif halométhylhalosilane **S**, tel que défini dans la 1^{ère} revendication, et ii) la réaction à une source d'ions fluorure, qui comprend, facultativement, plusieurs étapes intermédiaires.

8. Utilisation du réactif halométhylhalosilane S pour la préparation des composés des formules **II**, **IV**, **VI** ou les sels de celui-ci ou du Raltegravir ou des sels pharmaceutiquement acceptables de celui-ci, moyennant une méthode d'introduction selevtive d'un groupe de méthyl sur le groupement des hétérocycles-NH-, dans laquelle le réactif S, et les composés II, IV et VI sont tels que définis dans les revendications précédentes.
